# EUROPEAN PATENT SPECIFICATION

(11) **EP 3 663 391 B1**
(45) Date of publication and mention of the grant of the patent: **07.04.2021**
(21) Application number: 18000944.1
(22) Date of filing: 07.12.2018
(51) Int. Cl.: C12N 1/18, C12P 7/22, C12R 1/85

(54) **A STRAIN FOR THE PRODUCTION OF NATURAL TYROSOL AND A PREPARATION METHOD OF NATURAL TYROSOL**
STAMM ZUR HERSTELLUNG VON NATÜRLICHEM TYROSOL UND VERFAHREN ZUR HERSTELLUNG VON NATÜRLICHEM TYROSOL
SOUCHE POUR LA PRODUCTION DE TYROSOL NATUREL ET PROCÉDÉ DE PRÉPARATION DE TYROSOL NATUREL

(43) Date of publication of application: 10.06.2020
(73) Proprietor: Xiamen Oamic Biotechnology Co., Ltd., Xiamen (CN)
(72) Inventor: Zhao, Xijing, Xiamen (CN); Xing, Chenguang, Xiamen (CN); Liu, Wei, Xiamen (CN); Huang, Liuyun, Xiamen (CN); Chen, Zuqing, Xiamen (CN)
(74) Representative: Verscht, Thomas Kurt Albert

(56) References cited:
- SIMONA GUERRINI ET AL: "Impact of Saccharomyces cerevisiae Strains on Health-Promoting Compounds in Wine", FERMENTATION, vol. 4, no. 2, 9 April 2018 (2018-04-09), page 26, XP55587125, DOI: 10.3390/fermentation4020026
- HISAYOSHI SOEJIMA ET AL: "Breeding of a high tyrosol-producing sake yeast by isolation of an ethanol-resistant mutant from a trp3 mutant : Breeding of a high tyrosol-producing sake yeast", JOURNAL OF THE INSTITUTE OF BREWING., vol. 118, no. 3, 1 November 2012 (2012-11-01), pages 264-268, XP55586882, GB ISSN: 0046-9750, DOI: 10.1002/jib.46
- DATABASE WPI Week 200453 Thomson Scientific, London, GB; AN 2004-548241 XP002791125, & JP 2004 215644 A (JAPAN SCI&TECHNOLOGY AGENCY) 5 August 2004 (2004-08-05)
- JINGJIE JIANG ET AL: "Metabolic Engineering of Saccharomyces cerevisiae for High-Level Production of Salidroside from Glucose", JOURNAL OF AGRICULTURAL AND FOOD CHEMISTRY, vol. 66, no. 17, 19 April 2018 (2018-04-19), pages 4431-4438, XP55587104, US ISSN: 0021-8561, DOI: 10.1021/acs.jafc.8b01272

## Description

### Technical Field

The invention relates to a strain for the production of natural tyrosol and a preparation method of natural tyrosol.

### Background of Invention

Tyrosol is widely found in olive oil, soy sauce, raspberry and fermentation products, such as beer, fruit wine and Japanese rice wine. It is an important pharmaceutical and fragrance intermediate. It is also widely used in the synthesis of surface modifiers, information storage materials and food additives.

At present, the main production methods of tyrosol are chemical synthesis and plant extraction. The chemical synthesis method has the disadvantages of high environmental pollution, harsh process conditions, high requirements on equipment and low yield. Low content of tyrosol in plants and complex extraction limit the supply of tyrosol. The production capacity is far from meeting market demand.

Ehrlich F proposed the biosynthesis of tyrosol in 1911. Elsden S. R. et al. suggested in 1958 that tyrosol synthesis was produced by fermentation of yeast, bacteria and some fungi at a yield of about 200 mg/L. Guerrini S. et al. (Fermentation 2018, 4(2), 26), Soejima H et al. (JOURNAL OF THE INSTITUTE OF BREWING, vol. 118, no. 3, 1 November 2012, pages 264-268) and japanese patent JP 2004 215644 describe Saccharomyces cerevisiae strains that produce tyrosol from tyrosine in alcoholic fermentation. Mohammed A. S. and others used *Candida* for fermentation. The yield relative to tyrosine was about 50%. It did not reach the production level of g/L but stayed at the level of mg/L, which cannot be industrialized.

More and more people advocate natural products, rejecting the use of chemically synthesized chemicals, so it is imperative to study a biological method for the synthesis of tyrosol.

### Summary of the Invention

One of the purposes of the invention is to provide a yeast *Saccharomyces* sp. OMK-58, which has the ability to synthesize natural tyrosol. The strain has been preserved at the China Center for Type Culture Collection (CCTCC) on July 13, 2018. The preservation place is Wuhan University, Wuhan City, Hubei Province, China. The preservation number is CCTCC M 2018479.

Another purpose of the invention is to provide the application of the yeast *Saccharomyces* sp. OMK-58 for the production of natural tyrosol.

A further purpose of the invention is to provide a method for the production of natural tyrosol by using the yeast *Saccharomyces* sp. OMK-58 for production.

Wherein, the method of production is a microbial fermentation method.

Preferably, the fermentation substrate of the microbial fermentation method is tyrosine.

Preferably, the production process of natural tyrosol includes the following steps:
1) the yeast *Saccharomyces* sp. OMK-58 strain activation;
2) the seed culture;
3) the fermentation: the seed liquid cultured to logarithmic growth phase is inoculated into a fermentation culture medium at a volume ratio of 5-20% under sterile conditions; the fermentation culture medium in mass consists of 2.0-5.0 %sugar raw material, 0.2-0.5% ammonium sulfate, 0.05-0.1% magnesium sulfate, 0.2-0.5% potassium dihydrogen phosphate, 0.1-1.5% yeast extract powder and 0.5-2.0% tyrosine; the fermentation culture medium containing seed liquid is fermented, and the fermentation liquid containing tyrosol is obtained.

Preferably, the initial pH value of the fermentation culture medium is 5.0-8.0, the fermentation culture medium containing seed liquid is fermented for 48-72 hours under the conditions of 30-37 °C, 200-500 rpm rotational speed and 1:0.05-0.15 ventilation.

Preferably, the said sugar raw material is at least one of glucose, sucrose and starch.

Preferably, in step 3) tyrosine is added to the medium in one dose or in batches.

Preferably, when step 3) is followed by step 4), tyrosol is separated from the fermentation liquid.

Compared with the aforementioned technology, the technical solution has the following advantages:
The yeast provided by the invention can be used for the production of natural tyrosol by fermentation method. The tyrosine is used as a substrate during fermentation, and the target product is produced by microbial metabolism. It is safe at low temperature and low pressure, simple operation and less pollution, and is a safe and environmentally friendly production method.

### Brief Description of the Drawings

This invention is further explained in conjunction with the accompanying drawings and embodiments.
Fig. 1 is a chromatogram of tyrosol standard.
Fig. 2 is a chromatogram of tyrosine standard.
Fig. 3 is a chromatogram of fermentation liquid in embodiment 2.

### Detailed Description

### 1. The Strain

The experiment used baker's yeast as the starting strain to screen out a mutant strain by chemical mutagenesis. The mutant strain could transform L-tyrosine into tyrosol. The strain named *Saccharomyces* sp. OMK-58 was preserved in China Center for Type Culture Collection on July 13, 2018, under the preservation number CCTCC NO: M 2018479.

### 2. The strain culture

Under sterile conditions, a full inoculation loop of the strain liquid from the glycerol stock was uniformly spread on the solid slant medium, and then cultured in a biochemical incubator at 25-30 °C for 24-48 hours. The solid slant medium was wort medium.

### 3. The seed culture

Under sterile conditions, a full inoculation loop of well-groomed yeasts from the solid slant medium was taken and inserted into the sterile seed culture medium. The initial pH value of the seed culture medium was 5.0-8.0, and the strain was cultured to logarithmic growth phase at the culture temperature of 28-35 °C and rotation speed of 150-500 rpm. The seed culture medium formula by the mass ratio (%) is as follows:
1.0-3.5 one of the sugars such as glucose, sucrose or starch, 0.2-1.0 potassium dihydrogen phosphate, 0.1-0.5 dipotassium hydrogen phosphate, 0.2-0.8 sodium chloride, 0.1-1.0 yeast extract powder and 0.1-1.0 peptone.

### 4. The fermentation

The seed liquid cultured to exponential growth phase is used to inoculate a fermentation culture medium at a volume ratio of 10% under sterile conditions. The initial pH value of the fermentation culture medium is 5.0-8.0, and the optimum pH value is 5.0-6.0. The fermentation time is 48-72 hours under the conditions of 30-37 °C, 200-500 rpm rotational speed and 1:0.05-0.15 ventilation.

The fermentation formula by the mass ratio (%) is as follows:
2.0-5.0 one of the sugars such as glucose, sucrose and starch, 0.2-0.5 ammonium sulfate, 0.05-0.1 magnesium sulfate, 0.2-0.5 potassium dihydrogen phosphate, 0.1-1.5 yeast extract powder and 0.5-2.0 tyrosine.

Tyrosine can be added to the medium in one dose or in batches.

### 5. Determination

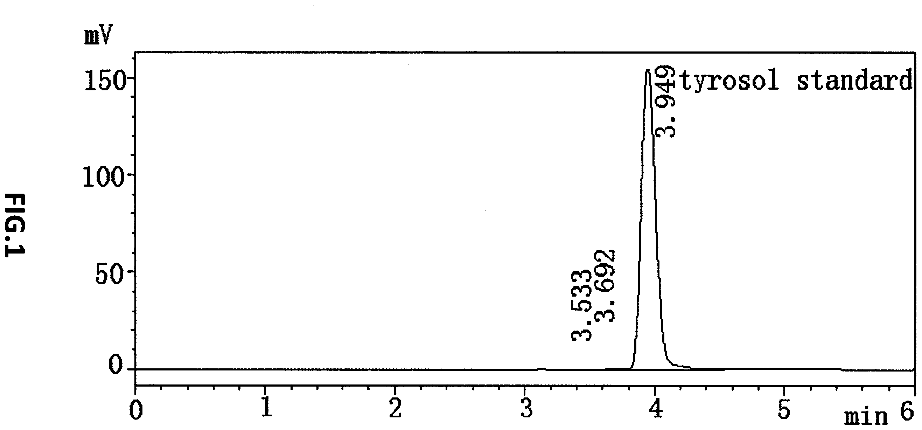

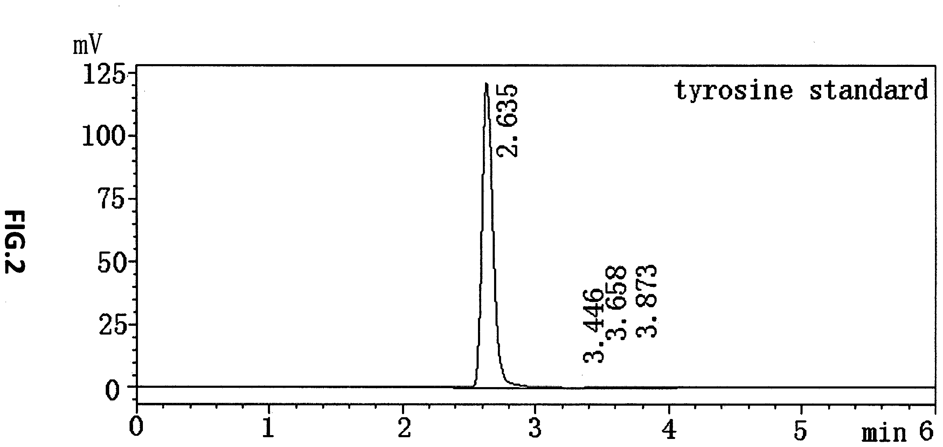

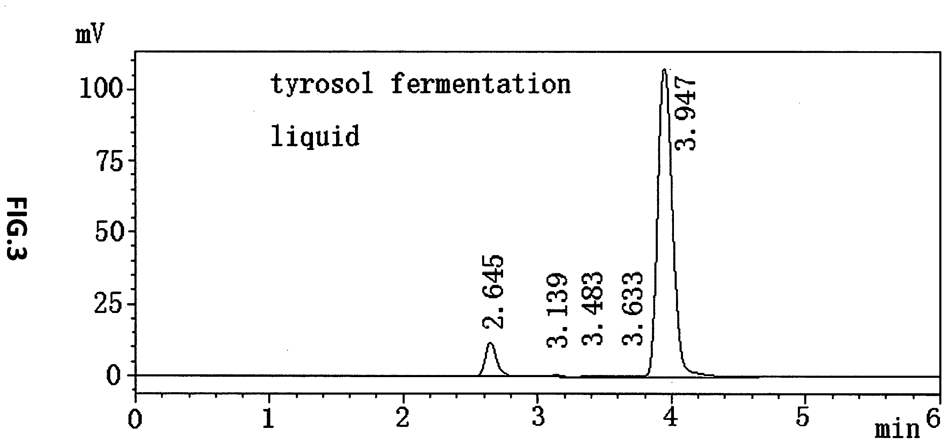

Determination method of tyrosol by high performance liquid chromatography (HPLC): chromatographic column: COSMOSIL 5C18-PAQ (4.6 ID*250 mm); mobile phase: acetonitrile: 0.15% phosphoric acid aqueous liquid = 70:30 (V/V); the flow rate is 0.8 mL/min; the detection wavelength was 216 nm; the column temperature is 35 °C. The injection volume is 5 µL, and the chromatogram is shown in Figures 1 to 3.

### Embodiment 1: the production of natural tyrosol (oscillating fermentation of triangular bottles)

The seed culture medium was prepared as follows (g/L): glucose 10, potassium dihydrogen phosphate 2.0, dipotassium hydrogen phosphate 1.0, sodium chloride 2.5, yeast extract powder 10.0, peptone 5.0 and water as the solvent; the initial pH was 6.0. The seed culture medium was sterilized at 121 °C for 15 minutes.

The fermentation culture medium was prepared as follows (g/L): glucose 30, potassium dihydrogen phosphate 4, ammonium sulfate 3.0, magnesium sulfate 0.8, yeast extract powder 1.0, tyrosine 10.0 and water as the solvent; the initial pH was 7.0. The fermentation culture medium was sterilized at 121 °C for 15 minutes.

The preparation of the seed: under sterile conditions, the strain of *Saccharomyces* sp. OMK-58 in low-temperature glycerol tube was transferred into a fresh, sterile solid plate for culture at 30 °C for 24 hours. A single colony of *Saccharomyces* sp. OMK-58 was picked and inoculated into a 500 mL flask for seed culture. The volume of the seed culture medium in the bottle was 50 mL. The flask was rotated at 180 rpm and cultured at 30 °C for 16 hours to obtain a seed liquid.

The fermentation of natural tyrosol: the fermentation culture medium of 50 mL was poured into a 500 mL sterile triangular flask, and then the 7.5 mL seed liquid was inoculated for fermentation. The fermentation temperature was 30 °C and the rotation speed of the shaker was 200 rpm. After fermentation for 100 hours, the concentration of natural tyrosol in fermentation liquid was determined by HPLC. The concentration was 2.0 g/L.

### Embodiment 2: the production of natural tyrosol (20 L bioreactor fermentation)

The seed culture medium and the fermentation culture medium were prepared in the same manner as embodiment 1.

The preparation of the seed: under sterile conditions, the strain of *Saccharomyces* sp. OMK-58 in low-temperature glycerol tube was transferred into a fresh, sterile solid plate for culture at 30 °C for 24 hours. A single colony was picked and inoculated into a 3 L seed tank containing 1.8 L seed culture medium for seed culture. The stirrer within the tank was rotated at 300 rpm and cultured at 30 °C for 18 hours to obtain the seed liquid.

The fermentation of natural tyrosol: the fermentation culture medium of 10.2 L was put into a 20 L fermentor, sterilized at 121 °C for 15 minutes, and then the 1.8 L seed liquid was inoculated into the 20 L fermentor for fermentation. The fermentation temperature was 30 °C, the rotation speed was 400 rpm, and the ventilation ratio was 1:0.1.. After fermentation for 60 hours, the concentration of natural tyrosol in fermentation liquid was determined by the HPLC method. The concentration was 5.0 g/L.

The invention may be summarized as follows: The invention discloses a strain for the production of natural tyrosol and a preparation method for natural tyrosol. The strain is a yeast, Saccharomyces sp. OMK-58. The strain can be used to produce natural tyrosol. The preparation method of natural tyrosol is to use the strain to produce tyrosol by microbial fermentation. The fermentation method includes steps of strain activation, seed culture, fermentation, extraction and so on. The method has the advantages of low temperature and low pressure, relatively safe and simple operation, and less pollution. The present method is a safe and environmentally friendly production method.

### Industrial Applicability

The invention adopts a fermentation method to produce natural tyrosol, with low temperature and low pressure, relatively safe and simple operation.

## Claims

1. A yeast *Saccharomyces* sp. OMK-58, wherein the strain is stored in China Center for Type Culture Collection with CCTCC NO: M 2018479.

2. The application of the yeast *Saccharomyces* sp. OMK-58 according to claim 1,-to produce natural tyrosol.

3. A method for the production of natural tyrosol, wherein the yeast *Saccharomyces* sp. OMK-58 according to claim 1 is used for production.

4. The method for the production of natural tyrosol according to claim 3, wherein the said method for production is a microbial fermentation method.

5. The method for the production of natural tyrosol according to claim 4, wherein the substrate for fermentation is tyrosine.

6. The method for the production of natural tyrosol according to claim 3, wherein the method comprises the following steps:
1) the yeast *Saccharomyces* sp. OMK-58 strain activation;
2) the seed culture;
3) the fermentation: the seed liquid cultured to logarithmic growth phase is inoculated into a fermentation culture medium with the volume ratio of 5-20% under sterile conditions; the fermentation culture medium in mass consists of 2.0-5.0% sugar raw material, 0.2-0.5% ammonium sulfate, 0.05-0.1% magnesium sulfate, 0.2-0.5% potassium dihydrogen phosphate, 0.1-1.5% yeast extract powder and 0.5-2.0% tyrosine; the fermentation culture medium containing seed liquid is fermented, and the fermentation liquid containing tyrosol is obtained.

7. The method for the production of natural tyrosol according to claim 6, wherein in step 2) the seed culture medium in mass includes: 1.0-3.5% sugar raw material, 0.2-1.0% potassium dihydrogen phosphate, 0.1-0.5% dipotassium hydrogen phosphate, 0.2-0.8% sodium chloride, 0.1-1.0% yeast extract powder and 0.1-1.0% peptone.

8. The method for the production of natural tyrosol according to claims 6 and/or 7, wherein the initial pH value of the fermentation culture medium is 5.0-8.0, the fermentation culture medium containing the seed liquid is fermented for 48-72 hours under the conditions of 30-37 °C, 200-500 rpm rotational speed and 1:0.05-0.15 ventilation.

9. The method for the production of natural tyrosol according to any one or more of the claims 6 to 8, wherein the said sugar raw material includes at least one of glucose, sucrose and starch.

10. The method for the production of natural tyrosol according to any one or more of claims 6 to 9, wherein in step 3) tyrosine is added to the medium in one dose or in batches.

## Patentansprüche

1. Eine Hefe Saccharomyces sp. OMK-58. wobei der Stamm im China Center for Type Culture Collection mit CCTCC NR: M 2018479 aufbewahrt wird.

2. Die Anwendung der Hefe Saccharomyces sp. OMK-58 nach Anspruch 1, um natürliches Tyrosol herzustellen.

3. Ein Verfahren für die Herstellung von natürlichem Tyrosol, wobei die Hefe Saccharomyces sp. OMK-58 nach Anspruch 1 für die Herstellung verwendet wird.

4. Das Verfahren für die Herstellung von natürlichem Tyrosol nach Anspruch 3, wobei das Verfahren für die Herstellung ein mikrobielles Fermentationsverfahren ist.

5. Das Verfahren für die Herstellung von natürlichem Tyrosol nach Anspruch 4, wobei das Substrat für die Fermentation Tyrosin ist.

6. Das Verfahren für die Herstellung von natürlichem Tyrosol nach Anspruch 3, wobei das Verfahren die folgenden Schritte aufweist:
1) die Hefe Saccharomyces sp. OMK-58 Stammaktivierung;
2) die Keimkultur;
3) die Fermentation: die Keimflüssigkeit, welche bis zur logarithmischen Wachstumsphase kultiviert wird, wird in ein Fennentationskultunnedium mit einem Volumenverhältnis von 5-20% unter sterilen Bedingungen geimpft; das Fermentationskulturmediums besteht nach Masse aus 2,0-5,0% Zuckerrohmaterial, 0,2-0,5% Ammoniumsulfat, 0,05-0,1% Magnesiumsulfat, 0,2-0,5% Kaliumdihydrogenphosphat, 0,1-1,5% Hefeextraktpulver und 0,5-2,0% Tyrosin; das Fermentationskulturmedium, welches die Keiniflüssigkeit enthält, wird fermentiert und die Fermentationsflüssigkeit, welche Tyrosol enthält, wird erhalten.

7. Das Verfahren für die Herstellung von natürlichem Tyrosol nach Anspruch 6, wobei in Schritt 2) das Keimkulturmedium nach Masse enthält: 1,0-3,5% Zuckerrohmaterial, 0,2-1,0% Kaliumdihydrogenphosphat, 0,1-0,5% Dikaliumhydrogenphosphat, 0,2-0,8% Natriumchlorid, 0,1-1,0% Hefeextraktpulver und 0,1-1,0% Pepton.

8. Das Verfahren für die Herstellung von natürlichem Tyrosol nach den Ansprüchen 6 und/oder 7, wobei der anfängliche pH-Wert des Fermentationskulturmediums 5,0-8,0 ist, wobei das Fermentationskulturmedium, welches die Keimflüssigkeit enthält, 48-72 Stunden unter den Bedingungen von 30-37 °C, 200-500 UpM Rotationsgeschwindigkeit und 1:0,05-0,15 Belüftung fermentiert wird.

9. Das Verfahren für die Herstellung von natürlichem Tyrosol nach einem oder mehreren der Ansprüche 6 bis 8, wobei das Zuckerrohmaterial wenigstens eines von Glucose, Saccharose und Stärke aufweist.

10. Das Verfahren für die Herstellung von natürlichem Tyrosol nach einem oder mehreren der Ansprüche 6 bis 9, wobei in Schritt 3) Tyrosin dem Medium in einer Dosis oder in Chargen zugegeben wird.

## Revendications

1. Levure *Saccharomyces* sp. OMK-58, dans laquelle la souche est stockée au Center for Type Culture Collection de Chine sous la référence CCTCC N° M 2018479.

2. Application de la levure *Saccharomyces* sp. OMK-58 selon la revendication 1 pour produire du tyrosol naturel.

3. Procédé pour la production de tyrosol naturel, dans lequel la levure *Saccharomyces* sp. OMK-58 selon la revendication 1 est utilisée pour la production.

4. Procédé pour la production de tyrosol naturel selon la revendication 3, dans lequel ledit procédé de production est un procédé de fermentation microbienne.

5. Procédé pour la production de tyrosol naturel selon la revendication 4, dans lequel le substrat pour la fermentation est la tyrosine.

6. Procédé pour la production de tyrosol naturel selon la revendication 3, dans lequel le procédé comprend les étapes suivantes :
1) l'activation de la souche de levure *Saccharomyces* sp. OMK-58 ;
2) la culture d'ensemencement ;
3) la fermentation : le liquide d'ensemencement cultivé jusqu'à la phase de croissance logarithmique est inoculé dans un milieu de culture par fermentation en un rapport en volume de 5 à 20 % dans des conditions stériles ; le milieu de culture par fermentation consiste, en masse, en 2,0 à 5,0 % de matière première de sucre, de 0,2 à 0,5 % de sulfate d'ammonium, de 0,05 à 0,1 % de sulfate de magnésium, de 0,2 à 0,5 % de dihydrogénophosphate de potassium, de 0,1 à 1,5 % d'extrait de levure en poudre et de 0,5 à 2,0 % de tyrosine ; le milieu de culture par fermentation contenant le liquide d'ensemencement est fermenté, et le liquide de fermentation contenant du tyrosol est obtenu.

7. Procédé pour la production de tyrosol naturel selon la revendication 6, dans lequel, dans l'étape 2), le milieu de culture d'ensemencement contient, en masse : 1,0 à 3,5 % de matière première de sucre, 0,2 à 1,0 % de dihydrogénophosphate de potassium, 0,1 à 0,5 % de dihydrogénophosphate dipotassique, 0,2 à 0,8 % de chlorure de sodium, 0,1 à 1,0 % d'extrait de levure en poudre et 0,1 à 1,0 % de peptone.

8. Procédé pour la production de tyrosol naturel selon les revendications 6 et/ou 7, dans lequel la valeur de pH initiale du milieu de culture par fermentation est de 5,0 à 8,0, le milieu de culture par fermentation contenant le liquide d'ensemencement est fermenté pendant 48 à 72 heures dans des conditions de 30-37°C, de vitesse de rotation de 200-500 t/min et de ventilation de 1/0,05-0,15.

9. Procédé pour la production de tyrosol naturel selon l'une quelconque ou plusieurs des revendications 6 à 8, dans lequel la matière première de sucre contient au moins l'un parmi le glucose, le saccharose et l'amidon.

10. Procédé pour la production de tyrosol naturel selon l'une quelconque ou plusieurs des revendications 6 à 9, dans lequel, dans l'étape 3), de la tyrosine est ajoutée au milieu en une seule fois ou par lots.
